# EUROPEAN PATENT SPECIFICATION

(11) **EP 1 322 315 B1**
(45) Date of publication and mention of the grant of the patent: **29.06.2011**
(21) Application number: 01972783.3
(22) Date of filing: 28.09.2001
(51) Int. Cl.: A61K 31/575

(54) **FORMULATIONS FOR REDUCING OR ELIMINATING TOXICITY OF ENVIRONMENTAL HORMONES CONTAINING URSODEOXYCHOLIC ACID**
FORMULIERUNGEN ZUR VERRINGERUNG ODER ELIMINIERUNG DER TOXIZITÄT VON UMWELTHORMONEN MIT URSODESOXYCHOLSÄURE
FORMULATIONS CONTENANT DE L'ACIDE URSODESOXYCHOLIQUE POUR REDUIRE OU SUPPRIMER LA TOXICITE DE MODULATEURS ENDOCRINIENS

(30) Priority: 04.10.2000 KR 2000058127
(43) Date of publication of application: 02.07.2003
(73) Proprietor: DAEWOONG PHARMACEUTICAL CO., LTD., Sungnam, Kyunggi-do 461-120 (KR)
(72) Inventor: YEON, Jae-Duck, Joongwon-ku, 462-152 Sungnam, Kyunggi-do (KR); BAIK, Kyong-Up, Joong-ku, 301-130 Taejeon (KR); JUNG, Kyu-Hyuck, 429-010 Siheung, Kyunggi-do (KR); PARK, Seung-Kook, Sanho Apt. 409, 462-120 Sungnam, Kyunggi-do (KR)
(74) Representative: Wichmann, Hendrik
(86) International application number: PCT/KR2001/001643
(87) International publication number: WO 2002/028396

(56) References cited:
- WO-A-01/47531
- WO-A-96/22771
- DE-A- 19 906 290
- KR-A- 2000 013 523
- KR-A- 2001 028 366
- US-A- 5 405 621
- US-A- 5 521 214
- DATABASE BIOSIS [Online] BIOSCIENCES INFORMATION SERVICE, PHILADELPHIA, PA, US; 24 September 1999 (1999-09-24) MITSUYOSHI HIRONORI ET AL: "Ursodeoxycholic acid protects hepatocytes against oxidative injury via induction of antioxidants" Database accession no. PREV199900504254 XP002267092 & BIOCHEMICAL AND BIOPHYSICAL RESEARCH COMMUNICATIONS, vol. 263, no. 2, 24 September 1999 (1999-09-24), pages 537-542, ISSN: 0006-291X
- DATABASE WPI Section Ch, Week 200107 Derwent Publications Ltd., London, GB; Class B04, AN 2001-057970 XP002275707 & KR 2000 013 523 A (KOREA GINSENG & TOBACCO RES INST), 6 March 2000 (2000-03-06)

## Description

### TECHNICAL FIELD

The present invention relates to formulations for reducing or eliminating toxicity of environmental hormones containing as a detoxifying agent ursodeoxycholic acid (3α, 7β-dihydroxy-5β-cholanoic acid; hereinafter, referred to as UDCA), or pharmaceutically acceptable salts or esters thereof.

### BACKGROUND ART

UDCA (the molecular weight: 392.58; the molecular formula: C₂₄H₄₀O₄) has various pharmacological effects *in vivo.* For example, it cleans bile canaliculi thereby to eliminate waste product and toxic bile acid therefrom, stabilizes hepatic cell membrane and protects hepatic cells. It also increases blood flow volume in liver, inhibits absorption and biosynthesis of cholesterol, dissolves gallstones and suppresses the formation thereof, and normalizes immune responses. Therefore, it is currently used for clinical treatment of cholelithiasis, biliary tract diseases, chronic hepatic diseases, hepatic insufficiency, post-enterectomy dyspepsia, fatty liver and the like. UDCA is an ingredient of animal bile, but present in human bile in extremely small quantities. Instead, CDCA (chenodeoxycholic acid), a stereoisomer of UDCA, is present in human bile, usually in the form of conjugate with glycine or taurine.

Environmental hormones are chemicals, which disrupt normal functions of endocrine system. They are released to the environment and absorbed into the body to have hormone-like functions *in vivo.* Thus, they are often called as endocrine disrupters. As the environmental hormones, 67 chemicals are currently registered in WWF (World Wildlife Fund) list, and 142 substances such as industrial chemicals, medicines and food additives, *etc*. are registered in Ministry of Health and Welfare in Japan. Endocrine disrupters registered in WWF list can be classified into 6 organic chlorine compounds including dioxin, 44 pesticides including DDT, 8 phthalates including butylbenzyl phthalate, 3 heavy metals including cadmium, bisphenol A and benzopyrene.

Such environmental hormones have various effects on organisms, *e.g*. masculinization as well as feminization, behavioral disorders, and tumor promotion, *etc*. Recently, they have been revealed by many researchers to have significant effects on wild ecosystem and to have similar effects on humans. Most of such endocrine disrupters exhibit toxicity *in vivo* even in extremely small quantities due to their estrogen-like activity and thus, are called as exogenous estrogen within environment.

In many countries including Korea, a wide variety of environmental hormones are detected from plastics, inner coating materials of aluminum cans, instant noodle containers, degradation products of detergents, exhaust gas from an incinerator, pesticides, *etc*. Extremely small as the amount exhausted is, they migrate between media and are biologically concentrated through food chain, due to their high lipophilicity and persistency within the environment. Thus, they have severely adverse effects on the ecosystem.

Among endocrine disrupters, DDT and bisphenol A are representatives which have estrogen-like activity *in vivo.* Bisphenol A is used as a solubilizer of polyvinyl chloride, a main ingredient in the manufacture of plastics, and contained at a large amount in plastic wares widely used at home. Therefore, humans are in a particularly high danger of being exposed to it. DDT and bisphenol A are known to exert effects on female reproductive organs, for example, to cause uterine hypertrophy Such uterine hypertrophy leads to the increase in uterus weight in animal experiments. Their known molecular biological mechanism is that they bind to estrogen receptor thereby to exhibit estrogen-like activity. Accordingly, DDT and bisphenol A exhibit the same effect on weight and shape of the uterus as estrogen in ovarian-ligated animals. Especially, halogenated aromatic hydrocarbon compounds such as polychlorinated benzene compounds and dioxin, the most noteworthy substances among the environmental hormones, are known to exhibit similar toxicity by common-reaction mechanism.

Halogenated aromatic hydrocarbons including polychlorinated dibenzodioxin and polychlorinated dibenzofuran are the most representative environmental pollutants exhausted from the burning of fossil fuels or the incineration of medical wastes, or in bleaching of paper. TCDD, the chemical name of which is 2,3,7,8-tetrachlorodibenzo-p-dioxin, is the most toxic compound among polychlorinated aromatic hydrocarbons displaying toxicity by common reaction mechanism. Humans are generally exposed to such compounds, which have been incorporated into foods, drinking water, soil, dust, smoke or air. In particular, laborers engaged in production, utilization or destruction of such substances encounter greater possibility of being continuously or intermittently exposed to a relatively high concentration of polyhalogenated aromatic hydrocarbons and their health is thus greatly threatened thereby. These compounds are biologically concentrated in fishes and then, fishers for living or hobby are orally exposed thereto. Farmers around incineration facilities may also be orally exposed thereto. In addition, they are released at a high concentration through industrial accidents and inappropriate disposal of industrial wastes. Volatile dioxin contained in ashes left after combustion in the incinerator is subject to the absorption into the lung. Dioxin is present in soil, air and sediment at an extremely low concentration, but may be biologically concentrated through food chain due to its lasting stability and lipophilicity. Humans are usually exposed to dioxin through foodstuff. The exposed concentration of dioxin to humans through foodstuff is supposed to be about 0.1-0.3 pg/kg/day.

The lipophilicity of dioxin and related substances leads to the enhancement of their concentrations in mother's milk and of release from adipose tissues in breast for a period of lactation. Thus, breast-fed newborns are daily exposed to about 10-20 fold higher concentration of dioxin. The precedent studies on blocking and detoxifying agents of dioxin revealed that it causes immunosuppression, endocrine disorders, carcinogenesis and ischemic cardiac diseases by binding to aromatic hydrocarbon receptor.

Casper R F., et al. (Mol. Phar. Macol. 1999 Oct.: 56(4): 784-790) reported that Resveratrol extracted from red' wine has the preventive effects on dioxin toxicity by binding to aromatic hydrocarbon receptor to which dioxin compounds are bound. As revealed by the precedent studies, Resveratrol has appropriate pharmacological effects and further, is a nontoxic substance. In the future, it will be thus developed as an agent for preventing dioxin toxicity after many clinical trials. However, although this compound has competitive inhibitory activities on dioxin having been previously absorbed into the body, it cannot suppress continuous absorption of dioxin *per se,* nor can it enhance the excretion thereof. Thus, the previously absorbed dioxin is accumulated in tissues, particularly, in liver or adipose tissues. Therefore, it is incapable of fully suppressing dioxin toxicity.

Additionally, olestra (Lancet, 1999, Oct. 9; 354(9186): 1266-1267) known as a substance having potential absorptive and excretory activities on dioxin is a polyfatty acid ester wherein many fatty acids are bound to sucrose and which cannot be degraded by pancreatic enzymes. This compound has a strong adsorptive activity on dioxin and accordingly, can efficiently excrete dioxin, which is reabsorbed via gastrointestinal circulation, and can block the reabsorption *per se* and thus, is very clinically useful. However, it also has the adsorptive activity on lipophilic vitamins, *e.g*. vitamins A, D, E, K and cartenoids and thus, has the drawback to interrupt the absorption of these essential vitamins.

In addition, activated carbon may be used as an adsorbent of dioxin, but has the difficulty in common use because of a low selectivity in adsorption, in the similar way to olestra.

Tamoxifen and Faslodex (ICI 182,780, AstraZeneca) known as inhibitors against estrogen or substances of analogous activity have been developed as therapeutic agents of breast cancer and are clinically tested in the present time. They may be clinically applied for detoxification of dioxin. However, tamoxifen currently used as the therapeutic agent of breast cancer has the side effect such as uterotropic activity and thus, has the limit in use as an agent for reducing the hazard of environmental hormones in humans. Based on results of the precedent studies, Faslodex reducing the side effect of tamoxifen used as the therapeutic agent of breast cancer has the excellent clinical efficacy. But its activity is limited to anticancer activity and its anti-estrogenic activity is substantially limited to only the competitive inhibitory action on estrogen receptor. Therefore, it can be clearly distinguished from UDCA having the eliminatory activity on *in vivo* load of estrogen and analogues of estrogenic activity.

### DISCLOSURE OF THE INVENTION

The present inventors found that UDCA increases biliary flow volume thereby to facilitate the excretion of toxic substances. Accordingly, an object of the present invention is to provide formulations as defined in the claims.

UDCA facilitates the excretion of toxic substances by increasing biliary flow volume. It also protects normal hepatic cells by efficient removal of dioxin, which is more readily accumulated in liver than any other organs. Simultaneously, it does not inhibit the absorption of essential lipophilic substances and thus, can detoxify them very effectively without any severe side effect.

Any toxicity, which may be caused by the exposure to various environmental hormones, can be ameliorated or eliminated by the use of UDCA or equivalents thereof. In the present invention, the equivalents of UDCA include any pharmaceutically acceptable salts or esters thereof known as having the equivalent pharmacological effects to UDCA in the art. Representative examples thereof are sodium salt of UDCA and taurine conjugate of UDCA.

As used herein, the term "environmental hormones" includes halogenated aromatic hydrocarbons such as PCB and dioxin compounds, and endocrine disrupters such as DDT, phthalates, alkyl phenol, bisphenol A, *etc.*

It was demonstrated that various toxic conditions caused by dioxin, *e.g*. death, weight loss, growth suppression, loss of blood glucose regulation, decrease in testicle weight, *etc*. can be suppressed or normalized by the administration of dioxin to experimental animals which were artificially exposed to dioxin. In addition, it was discovered that uterine hypertrophy by estrogen-like substances can be effectively inhibited by the use of UDCA. Based on the above, UDCA was confirmed to have anti-estrogenic effects *in vivo.*

TCDD was subcutaneously injected to mice administered with pharmacologically effective amount of UDCA, and after the given times, its detoxifying effect on TCDD was evaluated by measurement of changes in death rate, hematological test, organ extraction and immunostaining method. Then, in order to verify such effect, it was compared with that of activated charcoal, which has been known to block and eliminate TCDD toxicity continuously displayed through the enterohepatic circulation by inhibition of its absorption *in vivo.*

UDCA solubilizes in bile highly lipophilic substances such as DDT, phthalates, alkyl phenol, bisphenol A, *etc*., which display toxicity by continuous accumulation *in vivo,* thereby to efficiently excrete and detoxify them. Consequently, it can minimize *in vivo* effects of these substances.

Although experiments were carried out using TCDD, a representative of halogenated aromatic hydrocarbons, and bisphenol A the scope will not be limited to those specific substances, because any environmental hormones are known to exhibit similar toxicity by common reaction mechanism. Therefore, clinical trials using UDCA would be effective for detoxification, and suppression and prevention of toxicity from any other environmental hormones. Therefore, toxicity of environmental hormones can be effectively prevented or treated regardless of the kind or amount thereof.

The present formulations may contain pharmaceutically acceptable carriers conventionally used in the art and be manufactured into tablets, powder, granules, solutions, syrup, capsules, *etc*. in admixture with the pharmaceutically acceptable carriers. In addition, they may be manufactured into injectable solutions administered muscularly or intravenously. UDCA may be administered via any pharmaceutically acceptable routes.

According to this invention, UDCA is administered at an effective amount for suppressing toxicity by environmental hormones, preferably, 10 to 2,000 mg with a divided dose several times a day.

### BRIEF DESCRIPTION OF THE DRAWINGS

Fig. 1 is an optical microphotograph of seminiferous tubules in the solvent-administered control (x200).

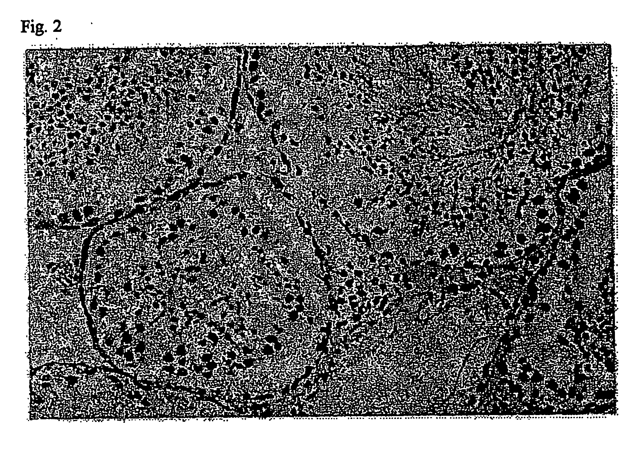

Fig 2 is an optical microphotograph of seminiferous tubules in the group administered with the dose of TCDD 27.5 µg/kg alone (×200). As shown in the figure, the remarkably reduced spermatogenesis was observed. Due to the disruption of cytoplasm, the shape of most cells was unclear. Reproductive endothelial cells in the basilar portion were considerably dissolved. In the seminiferous tubules of testes, separation of spermatogonia from the basilar portion was shown. There was a large gap between the basilar and lumenal portions. Normal spermatogenesis in seminiferous tubules was scarcely observed and seminiferous tubules having only Sertoli cells without reproductive cells were observed.
Fig 3 is an optical microphotograph of seminiferous tubules in the group co-administered with the dose of TCDD 27.5 µg/kg and 0.5% by weight of UDCA (x200). As shown in the figure, normal seminiferous tubules were observed, and some impaired seminiferous tubules and immature sperm cells were also observed in the center of seminiferous tubules.
Fig 4 is an electron microphotograph of Sertoli cells and reproductive cells in the solvent-administered control (x3,000). Sertoli cells were intimately contacted with reproductive cells and tight junction was observed.
Fig 5 is an electron microphotograph of Leydig's cells and cytoplasmic organelles (×2,500). Normal forms of Leydig's cells and organelles were well retained.
Fig 6 is an electron microphotograph of Leydig's cells in the group administered with the dose of TCDD 27.5 µg/kg alone (×4,000). Phagolysosomes were observed in the cytoplasm ofLeydig's cells.
Fig 7 is an electron microphotograph of Sertoli cells in the group administered with the dose of TCDD 27.5 µg/kg alone (×4,000). Large lipid droplets were observed in the cytoplasm of Sertoli cells, and cytoplasmic vacuoles and phagolysosomes were also observed.
Fig 8 is an electron microphotograph of heads of sperms in the solvent-administered control (×25,000). Normally developed heads of sperms were observed.
Fig 9 is an electron microphotograph of heads of sperms in the group administered with the dose of TCDD 27.5 µg/kg alone (×10,000). Heads of sperms were frequently observed in cytoplasmic vacuoles of Sertoli cells.
Fig 10 is an electron microphotograph of heads of sperms in the group co-administered with the dose of TCDD 27.5 µg/kg and 0.5% by weight of UDCA (×8,000). Normal shapes of sperm heads were retained.

### BEST MODE FOR CARRYING OUT THE INVENTION

This invention will be better understood from the following examples. However, one skilled in the art will readily appreciate the specific materials and results described are merely illustrative of, and are not intended to, nor should be intended to, limit the invention as described more fully in the claims, which follow thereafter.

### Example 1: Detoxifying effect of UDCA on TCDD

### 1. Experimental animals

Male C57B1/6J mice of 5 weeks old having the average weight of 18-22 g which had been bred in CRJ (Charles River in Japan) were purchased and used in this example as experimental animals. Those mice were acclimated under the constant breeding condition, *i.e*. the temperature of 24±1 °C, the humidity of 60±10% and the light and darkness cycle of 12 hours, for one week and thereafter, utilized in the experiment.

The Experimental animals were supplied with Purina powdered feed (purchased from Hae Eun Trade) and supplied with water ad libitum. All the animals were weighed twice a week during experimentation.

### 2. Preparation and administration of test materials

2,3,7,8-tetrachlorodibenzo-p-dioxin (TCDD) dissolved in toluene (purity of 95% or more as a result of gas chromatography analysis), manufactured by Chem. Services Inc. in USA, was volatilized with nitrogen to obtain powder. The obtained powder was dissolved in a mixed solvent containing acetone and corn oil with a volume ratio of 1:6. The resulting solution each containing 110, 27.5 and 6.9 µg/kg of TCDD was subcutaneously injected to mice at a volume of 5 ml per body weight. UDCA as a test material was incorporated into the powdered feed at an amount of 0.5, 0.25 and 0.125% by weight, respectively. The mice were supplied with the feed from 10 days before (pre-administration) to 60 days after administration of TCDD (post-administration).

### 3. Sampling and testing

Upon completion of the experiment for 60 days, the experimental animals were fasted for 24 hours and anesthetized with ether. Then, they were abdominally incised and blood was gathered from abdominal vena cava. They were sacrificed by the release of blood. Liver, kidneys, testes and epididymides were extracted and all the extracted organs were weighed. Only the testes were separately kept and observed under optical and electron microscopes. For biochemical finding test, serum was separated from blood with a centrifuge and the separated serum was analyzed with an automated blood biochemical analyzer, Dimension (Dupont, USA). Blood was put to another blood gathering bottle and then, analyzed with an automated blood cell analyzer, Celldyn (Abbott, USA).

### 4. Optical microscopy of testes

The extracted testes were fixed in 10% neutralized formalin buffer for 24 hours and washed with the flowing water. It was subsequently dehydrated with ethanol and cleared with xylene and then, embedded with paraffin. It was sectioned with microtome (microm HM 440E) to a thickness of 2-3 µm and then, double stained with hematoxylin and eosin to observe under an optical microscope.

### 5. Electron microscopy of testes

The extracted testes were soaked with a pre-fixative (2.5% glutaraldehyde) made from pH 7.3 phosphate buffer solution and sliced to a size of 1 mm³. It was pre-fixed in the pre-fixative at 4 °C for 4 hours and then, washed with the same buffer solution. Subsequently, it was fixed with 1% osmium tetroxide (pH 7.3) for 2 hours and washed with the same buffer. It was dehydrated with ethanol and substituted with propylene oxide, and embedded in epon mixed solution. The embedded tissue was prepared into ultra-thin section with a thickness of 60-70 nm using ultramicrotome (Reichert-Jung, Ultracut E) and then, double stained with uranyl acetate and lead citrate to observe under a Transmission Electron Microscope (H-600, Hitachi, Japan).

### 6. Determination of Johnsen's score

For evaluation of the level of spermatogenesis, Johnsen's score was determined from 10 to 1 based on the presence or absence of principal cells arranged according to maturation of spermatogenesis. The basis of Johnsen's score in testes was the following:
10: Perfect spermatogenesis having many sperms, organization of embryonic epithelial cells with a constant thickness, and opened lumen
9: Despite many sperms, significant separation of embryonic epithelial cells from the basement membrane and disruption thereof, or loss of lumen
8: Presence of 5 to less than 10 sperms in the section
7: Many sperm cells without sperms
6: 5 to less than 10 sperm cells without sperms
5: Several or many spermatocytes without sperms and sperm cells
4: Less than 5 spermatocytes without sperms and sperm cells
3: Presence of only spermatogonia as reproductive cells
2: Presence of only Sertoli cells without any reproductive cells
1: No cells in the section of seminiferous tubules

### 7. Statistics

The results are expressed as Mean±Standard Deviation values. For statistical analysis of the body weight, feed and water uptake, biochemical findings, hematological findings and the weight of organ in an acute toxicity test, verification was performed by one way analysis of variance (ANOVA) in case of equal distribution. In case significance was recognized in ANOVA, the Student's *t*-test was performed in the levels of p<0.05 and p<0.01.

### 8. Results

### (1) Effect on death rate and death time

**Table 1.**

| Effect of UDCA on death of mice by a single subcutaneous injection of TCDD | | | | |
|---|---|---|---|---|
| Dose (µ/kg) | Additive (wt%) | No. of dead mice/ No. of administered mice | Death rate (%) | Death time(days) (Mean±SD) |
| 0 | Solvent control | 0/10 | 0 | - |
| 6.9 | UDCA(0) | 0/10 | 0 | - |
| | UDCA(0.125) | 0/10 | 0 | - |
| | UDCA(0.25) | 0/10 | 0 | - |
| | UDCA(0.5) | 0/10 | 0 | - |
| 27.5 | UDCA(0) | 3/10 | 30 | 55±5 |
| | UDCA(0.125) | 0/10 | 0 | - |
| | UDCA(0.25) | 0/10 | 0 | - |
| | UDCA(0.5) | 0/10 | 0 | - |
| 110.0 | UDCA(0) | 10/10 | 100 | 35±7 |
| | UDCA(0.125) | 10/10 | 100 | 33±4 |
| | UDCA(0.25) | 9/10 | 90 | 34±5 |
| | UDCA(0.5) | 4/10 | 40 | 51±8 |
| | Activated C(0.5) | 7/10 | 70 | 35±6 |

- Group administered with the dose of TCDD 6.9 µg/kg: No animal died irrespective of the exposure to UDCA.
- Group administered with the dose of TCDD 27.5 µg/kg: Without exposure to UDCA, the death rate was 30% and the death time was 5.5±5 days, that is, animals died approximately in the end of experimentation. By contrast, no animal died with exposure to 0.125, 0.25 and 0.5% by weight of UDCA irrespective of its dose.
- Group administered with the dose of TCDD 110 µg/kg: Without exposure to UDCA, all the animals died on 35±7 days. With exposure to 0.125% by weight of UDCA, all the animals died on 33±4 days. With exposure to 0.25% by weight of UDCA, 90% of the animals died on 34±5 days. With exposure to 0.5% by weight of UDCA, 40% of the animals died on 51±8 days. In comparison, with exposure to 0.5% by weight of activated carbon, 70% of the animals died on 35±6 days.

### (2) Effect on changes in body weight

**Table 2.**

| Effect of UDCA on changes in body weight by a single subcutaneous injection of TCDD | | | | | | | | |
|---|---|---|---|---|---|---|---|---|
| Dose (µg/kg) | Additive (wt%) | Changes in body weight after administration of TCDD | | | | | | |
| | | 0 day | 12 days | 22 days | 32 days | 39 days | 50 days | 59 days |
| 0 | 0 | 20.67± 0.29 | 22.99± 0.42 | 24.54± 0.49 | 25.86± 0.58 | 27.15± 0.62 | 28.73± 0.62 | 29.28± 0.76 |
| 6.9 | 0 | 20.22± 0.31 | 22.27± 0.37 | 23.68± 0.36 | 24.37± 0.48 | 25.35± 0.66 | 27.03± 1.35 | 25.98± 1.44** |
| | UDCA | 19.77± | 21.95± | 23.16± | 24.15± | 25.21± | 26.62± | 27.06± |
| | (0.125) UDCA | 0.21 20.13± | 0.28 22.18± | 0.32 23.81± | 0.38 24.21± | 0.46 25.69± | 0.56 27.15± | 0.60 27.74± |
| | (0.25) UDCA (0.5) | 0.34 19.97± 0.28 | 0.38 21.81± 0.32 | 0.43 23.26± 0.37 | 0.47 24.28± 0.50 | 0.45 25.22± 0.49 | 0.46 27.15± 0.49 | 0.48 27.06± 0.56 |
| 27.5 | 0 | 19.54± 0.22 | 20.91± 0.52** | 19.82± 1.14** | 18.54± 1.16** | 17.80± 1.40** | 16.74± 1.22** | 15.72± 1.17** |
| | UDCA (0.125) | 19.80± 0.25 | 22.04± 0.26 | 23.59± 0.28 | 24.17± 0.33## | 25.48± 0.36## | 26.63± 0.28## | 26.93± 0.34## |
| | UDCA (0.25) | 19.96± 0.30 | 22.22± 0.29 | 23.56± 0.37# | 24.34± 0.38## | 25.38± 0.37## | 26.94± 0.50## | 27.27± 0.51## |
| | UDCA (0.5) | 19.08± 0.20 | 21.09± 0.32 | 22.52± 0.33 | 23.33± 0.32## | 24.07± 0.34## | 25.31± 0.43## | 24.52± 0.42## |
| 110.0 | 0 | 19.36± 0.31 | 19.96± 0.32** | 20.02± 0.47** | 19.84± 0.55** | 17.62± 1.14** | - | - |
| | UDCA (0.125) | 19.48± 0.29 | 19.40± 0.40 | 18.44± 0.87 | 12.61 | - | - | - |
| | UDCA (0.25) | 19.65± 0.44 | 20.64± 0.50 | 19.69± 1.03 | 18.78± 1.14 | 14.26 | 14.85 | 17.10 |
| | UDCA (0.5) | 19.71± 0.14 | 20.52± 0.32 | 21.15± 0.82 | 20.76± 1.04 | 19.81± 1.37 | 18.19± 1.80 | 18.53± 1.59 |
| | Activated C (0.5) | 20.10± 0.32 | 21.06± 0.35 | 20.46± 0.87 | 21.25± 1.20 | 20-54± 1.76 | 19.20± 0.77 | 18.28± 1.58 |

| | | | | | | | | |
|---|---|---|---|---|---|---|---|---|
| **: Significant difference from the solvent control in p<0.01 #: Significant difference from the group administered with TCDD alone in p<0.05 ##: Significant difference from the group administered with TCDD alone in p<0.01 | | | | | | | | |

- : Death of all the animals
- Group administered with the dose of TCDD 6.9 µg/kg: There was no statistically significant difference between the group without exposure to UDCA and the solvent control group. But, the weight was significantly decreased at 59 days after administration of TCDD. By contrast, such statistically significant weight loss was suppressed with exposure to 0.125, 0.25 and 0.5% by weight of UDCA.
- Group administered with the dose of TCDD 27.5 µg/kg: Without exposure to UDCA, the weight was continuously and significantly decreased compared with the solvent control group from 12 days after the administration of TCDD. However, with exposure to 0.125, 0.25 and 0.5% by weight of UDCA, such statistically significant weight loss was suppressed and the weight was maintained in the almost same level as the solvent control.
- Group administered with the dose of TCDD 110 µg/kg: Without exposure to UDCA, the weight was continuously and significantly decreased compared with the solvent control group from 12 days after the administration of TCDD. Further, with exposure to 0.125, 0.25 and 0.5% by weight of UDCA, the weight was continuously decreased in the same manner as the group without exposure to UDCA.

### (3) Uptake of feed and water

**Table 3.**

| Effect of UDCA on the feed uptake by a single subcutaneous injection of TCDD | | | | | | | |
|---|---|---|---|---|---|---|---|
| Dose (µg/kg) | Additive (wt%) | Changes in the feed uptake after administration of TCDD | | | | | |
| | | 10 days | 20 days | 30 days | 40 days | 50 days | 60 days |
| - | Solvent control | 4.61± 0.58 | 4.31± 0.58 | 4.44± 0.79 | 3.89± 0.44 | 3.90± 0.35 | 4.31± 0.95 |
| 6.9 | - | 4.76± 0.74 | 4.33± 0.84 | 4.97± 1.00 | 4.97± 1.00 | 3.51± 0.31 | 3.28± 1.56 |
| | UDCA/0.125 | 4.64± 0.73 | 4.55± 0.44 | 3.83± 0.36 | 3.83± 0.36 | 3.19± 0.13 | 2.59± 0.23 |
| | UDCA/0.25 | 4.39± 0.79 | 4.86± 0.90 | 3.78± 0.57 | 3.78± 0.57 | 3.18± 0.56 | 2.90± 0.30 |
| | UDCA/0.5 | 4.48± 0.67 | 4.00± 1.11 | 4.66± 0.67 | 4.66± 0.67 | 3.12± 0.40 | 3.35± 1.56 |
| 27.5 | - | 3.86± 0.87 | 3.17± 1.68 | 2.37± 0.37** | 1.90± 0.27** | 1.50± 0.16** | 1.58± 0.32** |
| | UDCA/0.125 | 4.15± 0.64 | 3.85± 0.43 | 3.99± 0.34## | 3.72± 0.21## | 2.90± 0.98## | 2.96± 0.29## |
| | UDCA/0.25 | 4.07± 0.40 | 3.41± 0.71 | 3.59± 0.38## | 3.32± 0.39## | 3.16± 0.24## | 3.10± 0.77## |
| | UDCA/0.5 | 4.31± 1.49 | 4.67± 1.13 | 3.99± 0.36## | 3.49± 0.57## | 3.18± 0.55## | 3.17± 0.49## |
| | - | 4.19± 0.96 | 3.21± 1.13 | 3.88± 0.96 | 2.23± 0.56 | - | - |
| | UDCA/0.125 | 3.77± 0.43 | 3.64± 1.16 | 2.94± 0.64 | 1.99± 0.58 | - | - |
| 110.0 | UDCA/0.25 | 3.65± 0.33 | 3.09± 0.44 | 3.23± 0.55 | 3.06± 1.89 | - | - |
| | UDCA/0.5 | 3.87± 0.57 | 3.77± 0.62 | 3.39± 0.24 | 2.72± 0.72 | 2.19± 0.22 | 2.05± 0.62 |
| | Activated C/ | 3.64± | 3.53± | 3.12± | 2.54± | 1.69± | 3.76± |
| | 0.5 | 0.52 | 0.24 | 0.47 | 0.33 | 0.33 | 1 1.79 |

| | | | | | | | |
|---|---|---|---|---|---|---|---|
| **: Significant difference from the solvent control in p<0.01 ##: Significant difference from the group administered with TCDD alone in P<0.01 | | | | | | | |

- : Death of all the animals
   As shown in the above Table 3, the feed uptake was remarkably reduced as the exposed concentration of TCDD was increased.
- Group administered with the dose of TCDD 6.9 µg/kg: No statistically significant difference was observed between the groups without and with exposure to UDCA.
- Group administered with the dose of TCDD 27.5 µg/kg: Statistically significant difference was observed between the groups without exposure to UDCA and with exposure to 0.125, 0.25 and 0.5% by weight of UDCA, from 30 days after administration of TCDD. That is, the decrease in feed uptake was suppressed by the administration of UDCA
- Group administered with the dose of TCDD 110 µg/kg: The feed uptake showed no statistically significant difference between the groups without and with exposure to UDCA.

**Table 4.**

| | | | | | | | |
|---|---|---|---|---|---|---|---|
| Effect of UDCA on the water uptake by a single subcutaneous injection of TCDD | | | | | | | |

| Dose (µg/kg) | Additive (wt%) | Changes in the water uptake after administration of TCDD | | | | | |
|---|---|---|---|---|---|---|---|
| | | 10 days | 20 days | 30 days | 40 days | 50 days | 60 days |
| - | Solvent | 4.39± | 4.36± | 4.82± | 5.36± | 6.25± | 7.07± |
| | control | 0.46 | 0.33 | 0.13 | 0.13 | 1.70 | 0.58 |
| 6.9 | - | 4.23± 0.52 | 4.08± 0.72 | 4.70± 0.42 | 4.30± 0.42** | 4.72± 0.39** | 4.88± 0.17** |
| | UDCA/0.125 | 4.54± 0.71 | 3.75± 0.90 | 4.00± 0.00 | 4.00± 0.00 | 4.06± 1.34 | 4.64± 0.51 |
| | UDCA/0.25 | 4.27± 1.16 | 3.83± 1.04 | 4.00± 0.00 | 3.60± 0.57 | 4.28± 1.02 | 4.69± 0.43 |
| | UDCA/0.5 | 4.12± 0.94 | 3.80± 0.70 | 4.00± 0.63 | 3.78± 0.31 | 4.35± 1.08 | 5.06± 0.70 |
| 27.5 | - | 3.38± 0.75 | 3.58± 0.14* | 3.10± 0.14** | 3.10± 0.14** | 2.89± 0.63** | 3.19± 0.21** |
| | UDCA/0.125 | 3.67± 1.10 | 3.92± 1.13 | 4.30± 0.42## | 4.20± 0.28## | 4.44± 0.79## | 4.81± 0.26## |
| | UDCA/0.25 | 4.04± | 3.92± | 4.00± | 4.00± | 4.44± | 4.81± |
| | UDCA/0.5 | 0.75 | 0.63 | 0.00## | 0.00## | 0.79## | 0.26## |
| | | 3.91± 0.95 | 4.35± 0.98 | 4.44± 0.00## | 4.44± 0.00## | 4.41± 1.00## | 4.88± 0.33## |
| 110.0 | - | 3.27± 0.78 | 4.00± 0.66 | 4.70± 0.42 | - | - | - |
| | UDCA/0.125 | 4.03± 1.21 | 4.07± 0.64 | 3.33± 0.94 | - | - | - |
| | UDCA/0.25 | 3.79± 1.06 | 3.83± 0.14 | 4.67± 0.94 | 4.14± 0.20 | - | - |
| | UDCA/0.5 | 3.75± 0.50 | 3.42± 0.80 | 4.20± 0.28 | 3.50± 0.71 | 3.14± 0.59 | 3.32± 0.33 |
| | Charcoal/ 2.5 | 4.21± 1.04 | 4.64± 0.47 | 4.70± 0.42 | 3.30± 0.990 | 7.67± 1.41 | 7.04± 0.44 |

| | | | | | | | |
|---|---|---|---|---|---|---|---|
| *: Significant difference from the solvent control in p<0.05 **: Significant difference from the solvent control in p<0.01 ##: Significant difference from the group administered with TCDD alone in p<0.01 | | | | | | | |

- : Death of all the animals
   As shown in the above Table 4, the water uptake was remarkably reduced as the exposed concentration of TCDD was increased.
- Group administered with the dose of TCDD 6.9 µg/kg: No statistically significant difference was observed between the groups without and with exposure to TCDD.
- Group administered with the dose of TCDD 27.5 µg/kg: Statistically significant difference was observed between the groups without exposure to UDCA and with exposure to 0.125, 0.25 and 0.5% by weight of UDCA, from 30 days after administration of TCDD. That is, the decrease in water uptake was suppressed by administration of UDCA
- Group administered with the dose of TCDD 110 µg/kg: The water uptake showed no statistically significant difference between the groups without and with exposure to UDCA.

### (4) Biochemical findings

For biochemical findings in the serum, the levels of glucose (GLU), GOT, GPT, Triglyceride (TG), high density lipoprotein (HDL-C), low density lipoprotein (LDL-C) and alkaline phosphatase (ALP) in serum were measured and the results are shown in the following.

**Table 5.**

| Effect of UDCA on the blood biochemical changes in mice by a single subcutaneous injection of TCDD | | | | | | | | |
|---|---|---|---|---|---|---|---|---|
| Dose (µg/kg) | Additive (wt%) | GLU (mg/dl) | GOT (IU/l) | GPT (IU/l) | TG (mg/dl) | HDL-C (mg/dl) | LDL-C (mg/dl) | ALP (IU/l) |
| - | Solvent control | 140.38± 19.48 | S2.00± 3.82 | 21.25± 5.87 | 81.38± 11.67 | 52.88± 4.91 | 35.75± 6.45 | 66.88± 9.95 |
| 6.9 | - | 96.00± 15.15** | 55.16± 6.65 | 21.66± 5.39 | 76.14± 14.03 | 39.29± 9.38** | 24.43± 11.81* | 69.67± 8.80 |
| | UDCA (0.125) | 128.00± 27.26# | 49.80± 2.74 | 23.50± 8.32 | 79.56± 11.29 | 45.67± 2.74 | 14.56± 6.28# | 66.78± 7.21 |
| | UDCA (0.25) | 104.60± 24.87 | 57.30± 4.83 | 24.00± 6.39 | 75.90± 11.55 | 46.30± 5.08 | 21.20± 5.25 | 72.05± 6.63 |
| | UDCA (0.5) | 125.50± 14.98## | 47.11± 1.9 | 18.78± 5.07 | 84.20± 7.66 | 44.83± 2.04 | 28.33± 4.63 | 85.24± 10.28 |
| 27.5 | - | 28.20± 8.23** | 230.40± 72.90** | 70.4± 23.46** | 92.67± 16.79 | 21.80± 6.76** | 43.80± 14.99 | 65.4± 19.05 |
| | UDCA (0.125) | 55.22± 9.34## | 75.22± 6.38## | 54.67± 23.28 | 98.78± 17.14 | 34.78± 4.35## | 2.40± 1.14## | 75.14± 18.33 |
| | UDCA (0.25) | 82.44± 13.04## | 58.67± 5.54## | 42.11± 22.05# | 97.00± 17.68 | 37.44± 3.64 | 4.29± 4.03## | 79.63± 6.07 |
| | UDCA (0.5) | 64.22± 9.35## | 96.67± 31.63## | 45.38± 21.74# | 133.33± 13.17## | 47.00± 7.66## | 15.33 3.35## | 80.57± 14.52 |

| | | | | | | | | |
|---|---|---|---|---|---|---|---|---|
| *: Significant difference from the solvent control in p<0.05 **: Significant difference from the solvent control in p<0.01 #: Significant difference from the group administered with TCDD alone in p<0.05 ##: Significant difference from the group administered with TCDD alone in p<0.01 | | | | | | | | |

GLU was significantly decreased in proportion to the administration dose of TCDD. In each administration dose of TCDD, the exposure to 0.125, 0.25 and 0.5% by weight of UDCA displayed remarkable inhibitory effect on the decrease in the levels of glucose in serum. In the group administered with the dose of TCDD 27.5 µg/kg, GOT was significantly increased in comparison with the solvent control and the exposure to 0.125, 0.25 and 0.5% by weight of UDCA remarkably suppressed such increase. However, in the group administered with the dose of TCDD 6.9 µg/kg, GOT was not significantly increased compared with the solvent control. In the group administered with the dose of TCDD 27.5 µg/kg, GPT was significantly increased in comparison with the solvent control and the exposure to 0.25 and 0.5% by weight of UDCA remarkably suppressed such increase. HDL-C was significantly decreased in proportion to the administration dose of TCDD and the exposure to 0.125, 0.25 and 0.5% by weight of UDCA remarkably suppressed such decrease. In the group administered with the dose of TCDD 27.5 µg/kg, LDL-C was not significantly decreased in comparison with the solvent control and the exposure to 0.125, 0.25 and 0.5% by weight of UDCA resulted in the significant difference. In the group administered with the dose of TCDD 6.9 µg/kg, LDL-C was significantly decreased in comparison with the solvent control and the exposure to 0.125% by weight of UDCA resulted in the significant difference. TG and ALP had no significant change.

### (5) Hematological findings

For hematological findings, white blood cell (WBC), neutrophil (NEU), lymphocyte (LYM), monocyte (MONO), eosinophil (EOS), basophil (BASO) and red blood cell (RBC) counts, and hemoglobin concentration (HGB), hematocrit (HCT), mean cell volume (MCV), mean corpuscular hemoglobin (MCH), mean corpuscular hemoglobin concentration (MCHC), red blood cell distribution width (RDW) and platelet count (PLT) were measured and the results are as follows.

**Table 6**

| Effect of UDCA on the hematological changes by a single subcutaneous injection of TCDD in mice | | | | | | | | |
|---|---|---|---|---|---|---|---|---|
| Dose (µg/kg) | Additive (wt%) | WBC (10³) | NEU (10³) | LYM (10³) | MONO (10³) | EOS (10³) | BASO (10³) | RBC (10⁶) |
| - | Solvent control | 6.29± 2.06 | 0.36± 0.11 | 5.72± 1.92 | 0.06± 0.05 | 0.02± 0.04 | 0.14± 0.09 | 9.74± 0.24 |
| 6.9 | - | 5.94± 1.85 | 0.40± 0.24 | 4.27± 2.05 | 0.10± 0.07 | 0.01± 0.01 | 0.21± 0.12 | 9.29± 0.85 |
| | UDCA (0.125) | 5.55± 1.91 | 0.32± 0.13 | 5.06± 1.70 | 0.05± 0.06 | 0.004± 0.005 | 0.12± 0.07 | 9.66± 0.24 |
| | UDCA | 5.68± | 0.28± | 5.17± | 0.04± | 0.003± | 0.09± | 9.42± |
| | (0.25) | 1.50 | 0.07 | 1.49 | 0.02 | 0.002 | 0.04 | 0.37 |
| | UDCA (0.5) | 5.76± 1.13 | 0.34± 0.11 | 5.12± 1.13 | 0.04± 0.03 | 0.002± 0.002 | 0.10± 0.06 | 9.64± 0.26 |
| 27.5 | - | 4.29± 1.05 | 2.58± 0.97** | 1.43± 0.60** | 0.07± 0.04 | 0018± 0.028 | 0.17± 0.07 | 9.10± 0.71 |
| | UDCA (0.125) | 3.69± 0.72 | 0.34± 0.10## | 2.06± 0.81 | 0.02± 0.01 | 0.002± 0.003 | 0.09± 0.04 | 9.61± 0.29 |
| | UDCA (0.25) | 3.78± 1.19 | 0.48± 0.09## | 2.9± 1.06## | 0.05± 0.03 | 0.008± 0.007 | 018± 0.07 | 9.48± 0.29 |
| | UDCA (0.5) | 3.71± 0.99 | 0.59± 0.23## | 3.04± 0.77## | 0.05± 0.04 | 0.005± 0.003 | 0.14± 0.07 | 9.57± 0.25 |
| Dose (µg/kg) | Additive (wt%) | HGB (g/dl) | HCT (%) | MCV (fl) | MCH (pg) | MCHC (g/dl) | RDW (%) | PLT (10³) |
| - | Solvent control | 14.60± 0.35 | 46.00± 1.32 | 47.20± 0.81 | 14.98± 0.20 | 31.73± 0.56 | 20.27± 1.00 | 1073.80± 69.74 |
| 6.9 | - | 13.65± 1.34* | 43.12± 5.04 | 46.33± 1.97 | 14.70± 0.41 | 31.76± 0.70 | 2038± 1.21 | 1140.88± 133.39 |
| | UDCA (0.125) | 14.35± 0.37 | 44.61± 1.14 | 46.18± 0.55 | 14.87± 0.22 | 32.18± 0.43 | 20.17± 1.07 | 1117.7± 88.65 |
| | UDCA (0.25) | 14.16± 0.44 | 44.77± 1.57 | 47.56± 0.81 | 15.04± 0.25 | 31.63± 0.58 | 19.73± 0.91 | 1141.22± 67.79 |
| | UDCA (0.5) | l4.47± 0.29 | 45.35± 0.93 | 47.08± 0.63 | 14.98± 0.19 | 31.87± 0.41 | 20.19± 0.72 | 1112.89± 76.53 |
| 27.5 | - | 12.90± 1.00** | 39.12± 3.25** | 42.97± 0.56** | 14.17± 0.40** | 33.02± 0.86** | 20.97± 2.27 | 1473.17± 110.35** |
| | UDCA (0.125) | 14.64± 0.67## | 43.77± 1.37## | 45.51± 0.35## | 15.32± 0.39## | 33.65± 1.04 | 20.08± 1.20 | 1172.44± 80.58## |
| | UDCA (0.25) | 14.23± 0.55## | 43.65± 1.24## | 46.09± 0.42## | 15.01± 0.44## | 32.58± 0.73 | 19.99± 1.08 | 1069.70± 128.48## |
| | UDCA (0.5) | 14.80± 0.78## | 44.49± 1.63## | 46.46± 0.86## | 15.46± 0.59## | 33.23± 1.04 | 20.44± 0.82 | 1068.14± 84.28## |

| | | | | | | | | |
|---|---|---|---|---|---|---|---|---|
| *: Significant difference from the solvent control in p<0.05 **: Significant difference from the solvent control in p<0.01 ##: Significant difference from the group administered with TCDD alone in p<0.01 | | | | | | | | |

WBC, MONO, EOS, BASO, RBC and RDW were not significantly changed by the administration of TCDD. NEU was significantly increased compared with the solvent control in the group administered with the dose of TCDD 27.5 µg/kg and the exposure to 0.125, 0.25 and 0.5% by weight of UDCA remarkably suppressed such increase. LYM was significantly decreased compared with the solvent control in the group administered with the dose of TCDD 27.5 µg/kg and the exposure to 0.25 and 0.5% by weight of UDCA remarkably suppressed such decrease. HGB was significantly decreased compared with the solvent control in the group administered with the dose of TCDD 27.5 µg/kg and the exposure to 0.125, 0.25 and 0.5% by weight of UDCA remarkably suppressed such decrease. HCT was significantly decreased compared with the solvent control in the group administered with the dose of TCDD 27.5 µg/kg and the exposure to 0.125, 0.25 and 0.5% by weight of UDCA remarkably suppressed such decrease. MCV was significantly decreased compared with the solvent control in the group administered with the dose of TCDD 27.5 µg/kg and the exposure to 0.125, 0.25 and 0.5% by weight of UDCA remarkably suppressed such decrease. MCH was significantly decreased compared with the solvent control and the exposure to 0.125, 0.25 and 0.5% by weight of UDCA remarkably suppressed such decrease. MCHC was significantly decreased compared with the solvent control in the group administered with the dose of TCDD 27.5 µg/kg and the exposure to UDCA could not suppress such decrease. PLT was significantly increased compared with the solvent control in the group administered with the dose of TCDD 27.5 µg/kg and the exposure of 0.125, 0.25 and 0.5% by weight of UDCA remarkably suppressed such increase. PLT was not significantly changed compared with the solvent control in the group administered with the dose of TCDD 6.9 µg/kg.

### (6) Measurement of organ weights

The organs, *i.e*. liver, kidneys, testes and epididymides, were weighed and the results are shown in the following.

**Table 7.**

| Effect of UDCA on changes in body weight, and weight and relative weight of liver by a single subcutaneous injection of TCDD in mice | | | | |
|---|---|---|---|---|
| Dose (µg/kg) | Additive (wt%) | Weight (g) | Liver | |
| | | | Weight(g) | Liver Relative weight(%) |
| - | Solvent control | 26.88±2.11 | 0.966±0.049 | 3.605±0.181 |
| 6.9 | - | 22.96±3.97* | 0.935±0.173 | 4.092±0.403** |
| | UDCA/0.125 | 24.12±1.83 | 0.980±0.073 | 4.069±0.167 |
| | UDCA/0.25 | 24.72±1.39 | 0.954±0.057 | 3.870±0.289 |
| | UDCA/0.5 | 24.19±1.38 | 1.028±0.073 | 4.258±0.327 |
| 27.5 | - | 16.18±2.51** | 0.734±0.151** | 4506±0.304** |
| | UDCA/0.125 | 24.58±1.07## | 1.187±0.086## | 4.832±0.327 |
| | UDCA/0.25 | 24.62±1.44## | 1.088±0.030## | 4.428±0.198 |
| | UDCA/0.5 | 22.15±1.14## | 1.057±0.069## | 4.773±0.250 |
| 110.0 | UDCA/0.125 | 15.93 | 0.8153 | 5.118 |
| | UDCA/0.25 | 18.53±3.79 | 1.379±0.327 | 8.600±3.276 |
| | Charcoal/2.5 | 17.36±4.99 | 1.066±0.319 | 6.145±0.880 |

| | | | | |
|---|---|---|---|---|
| *: Significant difference from the solvent control in p<0.05 **: Significant difference from the solvent control in p<0.01 ##: Significant difference from the group administered with TCDD alone in p<0.01 | | | | |

In the group administered with the dose of TCDD 27.5 µg/kg, the weight of liver was significantly decreased and its relative weight was significantly increased, compared with the solvent control. Such decrease in the weight was remarkably suppressed by the exposure to 0.125, 0.25 and 0.5% by weight of UDCA, but the increase in the relative weight was not suppressed thereby. In the group administered with the dose of TCDD 6.9 µg/kg, only the relative weight was significantly increased compared with the solvent control. Such increase was not suppressed by the exposure to UDCA.

**Table 8.**

| Effect of UDCA on changes in weight and relative weight of kidneys by a single subcutaneous injection of TCDD in mice | | | | | |
|---|---|---|---|---|---|
| Dose (µg/kg) | Additive (wt%) | Left kidney | | Right kidney | |
| | | Relative Weight(g) | weight(%) | Weight(g) | Relative weight(%) |
| - | Solvent control | 0.133±0.013 | 0.495±0.033 | 0.130±0.012 | 0.48S±0.046 |
| 6.9 | - | 0.115±0.018 | 0.508±0.050 | 0.117±0.022 | 0.513±0.066 |
| | UDCA(0.125) | 0.116±0.014 | 0.480±0.050 | 0.123±0.008 | 0.511±0.049 |
| | UDCA(0.25) | 0.119±0.009 | 0.484±0.036 | 0.123±0.013 | 0.500±0.048 |
| | UDCA(0.5) | 0.121±0.007 | 0.500±0.025 | 0.125±0.009 | 0.316±0.043 |
| 27.5 | - | 0.101±0.012** | 0.625±0.037** | 0.092±0.012** | 0.571±0.030** |
| | UDCA(0.125) | 0.127±0.012## | 0.517t0.056## | 0.120±0.013## | 0.489±0.055## |
| | UDCA(0.25) | 0.125±0.010## | 0.508±0.038## | 0.123±0.007## | 0.508±0.029## |
| | UDCA(0.5) | 0.119±0.011## | 0.539±0.040## | 0.119±0.009## | 0.338±0.045## |
| 110.0 | UDCA(0.25) | 0.1069 | 0.6711 | 0.0963 | 0.6045 |
| | UDCA(0.5) | 0.121±0.022 | 0.704±0.199 | 0.113±0.010 | 0.659±0.133 |
| | Act. C(0.5) | 0.113±0.031 | 0.652±0.017 | 0.107±0.025 | 0.622±0.017 |

| | | | | | |
|---|---|---|---|---|---|
| **: Significant difference from the solvent control in p<0.01 ##: Significant difference from the group administered with TCDD alone in p<0.01 | | | | | |

In the group administered with 27.5 µg/kg of TCDD, the weights of kidneys were significantly decreased and their relative weights were significantly increased, compared with the solvent control. Such changes were suppressed by the exposure to UDCA. In the group administered with 6.9 µg/kg of TCDD, no significant change was observed compared with the solvent control.

**Table 9.**

| Effect of UDCA on changes in weight and relative weight of testes by a single subcutaneous injection of TCDD in mice | | | | | |
|---|---|---|---|---|---|
| Dose (µg/kg) | Additive (wt%) | Left testis | | Right testis | |
| | | Weight(g) | Relative weigth(%) | Weight(g) | Relative weigth(%) |
| - | Solvent control | 0.076±0.006 | 0.283±0.024 | 0.075±0.005 | 0.281±0.023 |
| 6.9 | | 0.067±0.011* | 0.296±0.030 | 0.65±0.010** | 0.285±0.035 |
| | UDCA(0.125) | 0.072±0.006 | 0.297±0.020 | 0.073±0.007# | 0.304±0.020 |
| | UDCA(0.25) | 0.071±0.008 | 0.287±0.030 | 0.072±0.008 | 0.290±0.030 |
| | UDCA(0.5) | 0.075±0.010 | 0.310±0.050 | 0.074±0.007# | 0.307±0.031 |
| 27.5 | - | 0.058±0.014** | 0.356±0.051** | 0.058±0.015** | 0.3S2±0.059** |
| | UDCA(0.125) | 0.070±0.005# | 0.284±0.025## | 0.070±0.003# | 0.285±0.016## |
| | UDCA(0.25) | 0.072±0.007# | 0.293±0.032## | 0.071±0.007 | 0.290±0.030# |
| | UDCA(0.5) | 0.067±0.011 | 0.304±0.040# | 0.067±0.007 | 0.301±0.030# |
| 110.0 | UDCA(0.25) | 0.343 | 0.2153 | 0.363 | 0.2279 |
| | UDCA(0.5) | 0.057±0.019 | 0.316±0.074 | 0.057±0.019 | 0.314±0.067 |
| | Act. C(0.5) | 0.057±0014 | 0.332±0.037 | 0.056±0.016 | 0.326±0.22 |

| | | | | | |
|---|---|---|---|---|---|
| *: Significant difference from the solvent control in p<0.05 **: Significant difference from the solvent control in p<0.01 #: Significant difference from the group administered with TCDD alone in p<0.05 ##: Significant difference from the group administered with TCDD alone in p<0.01 | | | | | |

In the group administered with the dose of TCDD 27.5 µg/kg, the weights of testes were significantly decreased and their relative weight were significantly increased, compared with the solvent control. Such changes were suppressed by the exposure to UDCA to a variable extent depending upon concentrations of UDCA. In the group administered with the dose of TCDD 6.9 µg/kg, only the weight was significantly decreased compared with the control. Such decrease was suppressed by the exposure to 0.125 and 0.5% by weight of UDCA.

**Table 10.**

| Effect of UDCA on changes in weight and relative weight of epididymides by a single subcutaneous injection of TCDD in mice | | | | | |
|---|---|---|---|---|---|
| Dose (µg/kg) | Additive (wt%) | Left epididymis | | Right epididymis | |
| | | Weight(g) | Relative weight(%) | Weight(g) | Relative weight(%) |
| - | Solvent control | o.029±0.002 | 0.107±0.007 | 0.029±0.004 | 0.108±0.012 |
| 6.9 | - | 0.023±0.005** | 0.101±0.012 | 0.025±0.007 | 0.110±0.027 |
| | UDCA(0.125) | 0.029±0.003## | 0.121±0.011## | 0.029±0.003 | 0.121±0.014 |
| | UDCA(0.25) | 0.030±0.003## | 0.121±0.011## | 0.029±0.002 | 0.115±0.009 |
| | UDCA(0.5) | 0.029±0.003## | 0.121±0.010## | 0.027±0.004 | 0.114±0.015 |
| 27.5 | - | 0.021±0.006** | 0.124±0.024* | 0.021±0.006** | 0.129±0.027* |
| | UDCA(0.125) | 0.027±0.003# | 0.112±0.017 | 0.026±0.003 | 0.105±0.015# |
| | UDCA(0.25) | 0.029±0.003## | 0.120±0.018 | 0.030±0.005## | 0.124±0.020 |
| | UDCA(0.5) | 0.025±0.004 | 0.115±0.015 | 0.029±0.011 | 0.132±0.041 |
| 110.0 | UDCA(0.25) | 0.0195 | 0.122 | 0.0128 | 0.080 |
| | UDCA(0.5) | 0.021±0.007 | 0.114±0.025 | 0.024±0.009 | 0.127±0.024 |
| | Act. C(0.5) | 0.022±0.007 | 0.128±0.017 | 0.030±0.011 | 0.152±0.015 |

| | | | | | |
|---|---|---|---|---|---|
| *: Significant difference from the solvent control in p<0.05 **: Significant difference from the solvent control in p<0.01 #: Significant difference from the group administered with TCDD alone in p<0.05 ##: Significant difference from the group administered with TCDD alone in p<0.01 | | | | | |

In the group administered with the dose of TCDD 27.5 µg/kg, the weights of epididymides were significantly decreased and their relative weights were significantly increased, compared with the solvent control. Such changes were suppressed by the exposure to UDCA to a variable extent depending upon concentrations of UDCA. In the group administered with the dose of TCDD 6.9 µg/kg, only the weight of left epididymis was significantly decreased compared with the control and such decrease was suppressed by the exposure to UDCA. The relative weights were significantly increased compared with the group administered with TCDD alone.

### 7) Histopathological findings under an optical microscope

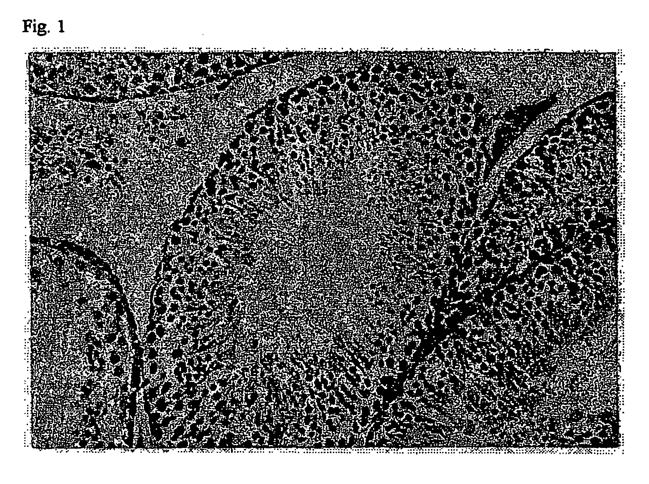

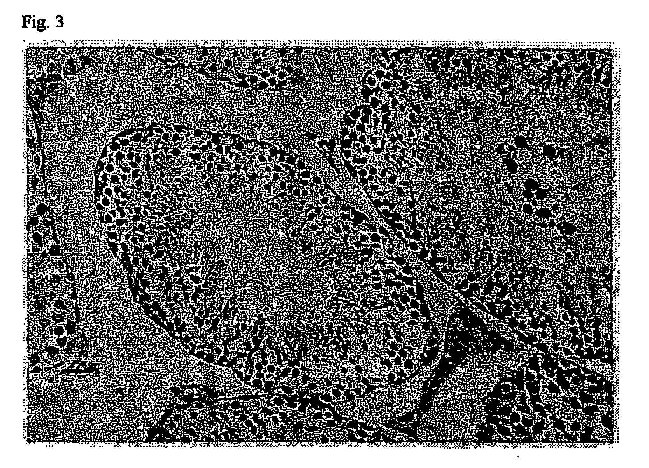

In the group administered with TCDD alone, most reproductive cells including spermatogonia showed the decreased differentiation. Due to the dissolution of reproductive epithelial cells, the separation of spermatogonia around basement membrane and the expansion of space between neighboring cells were observed. The expansion of space between basement section and lumen (inner space adjacent section) was also found. Further, growth arrest of spermatocytes and sperm cells was observed. In some groups, necrotic disease regions and seminiferous tubules having only Sertoli cells were observed. Therefore, TCDD was shown to cause very severe changes. By contrast, in the group exposed to UDCA, seminiferous tubules were found to have the similar shape to that of the solvent control and in some cases, spermatogenesis was conversely increased (see Figs 1, 2 and 3).

### 8) Changes in cellular microstructures under an electron microscope

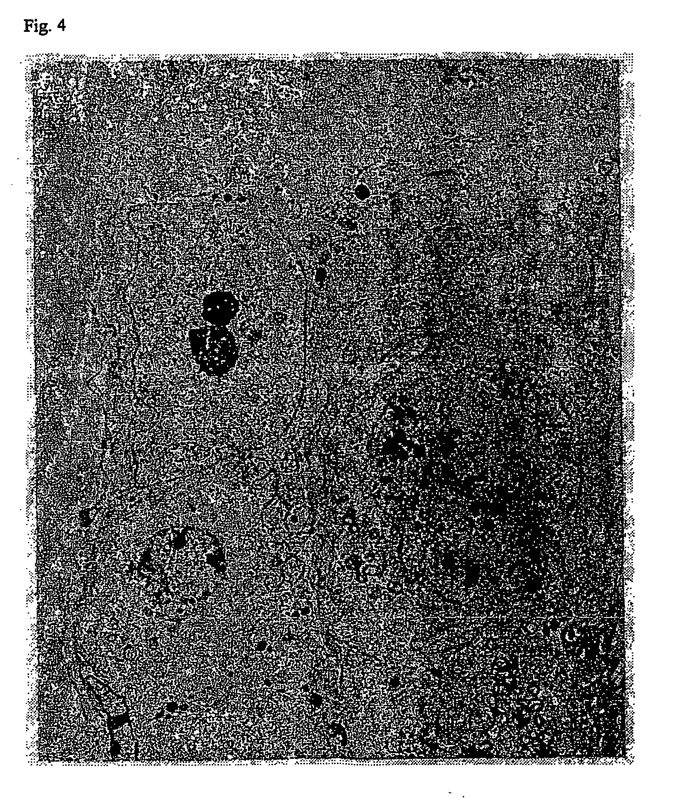

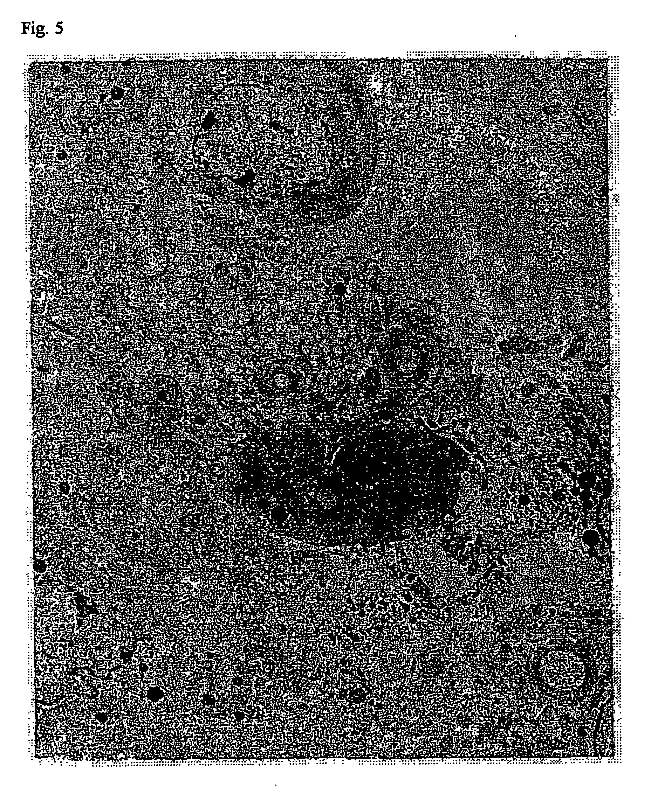

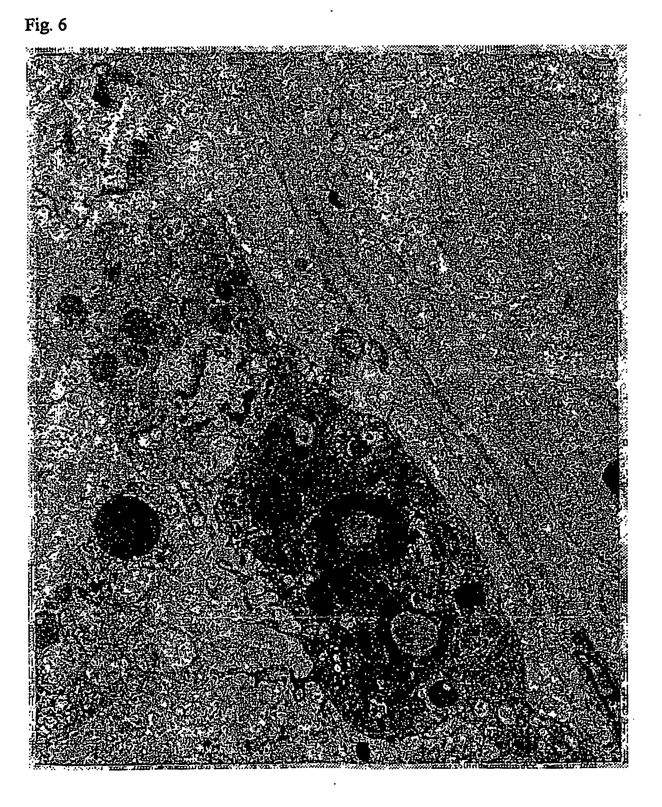

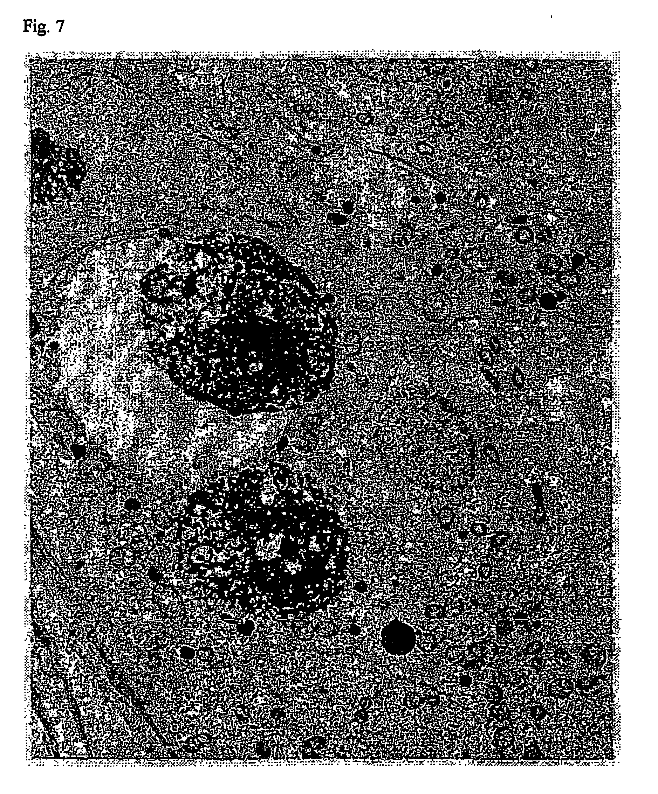

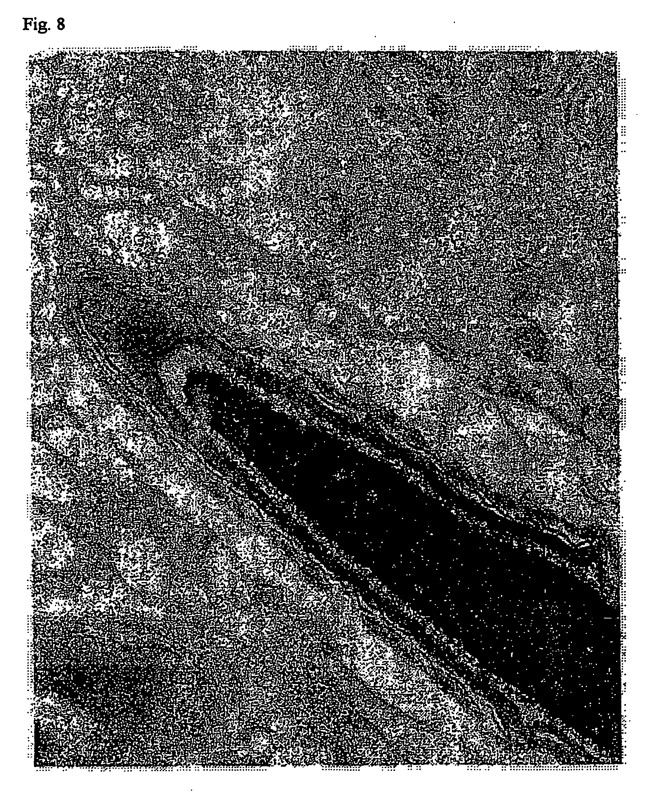

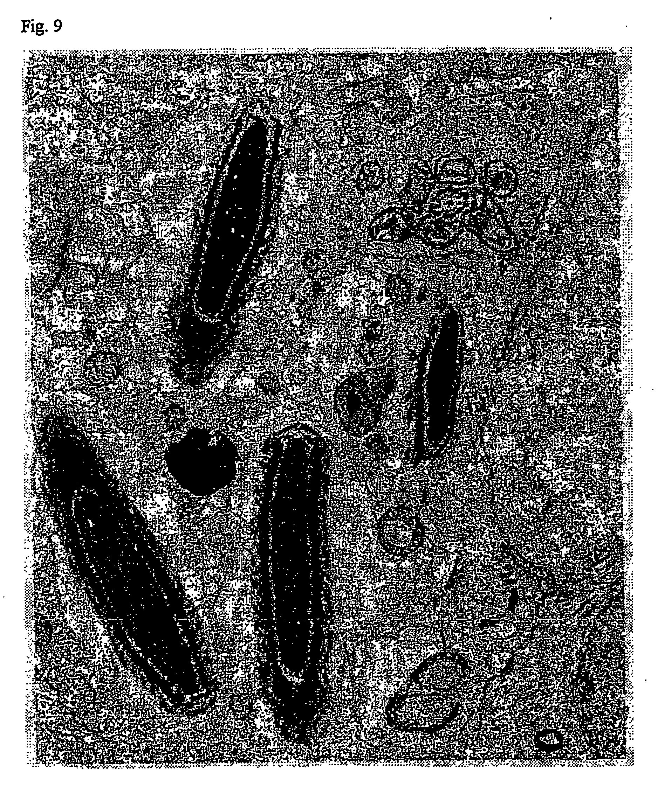

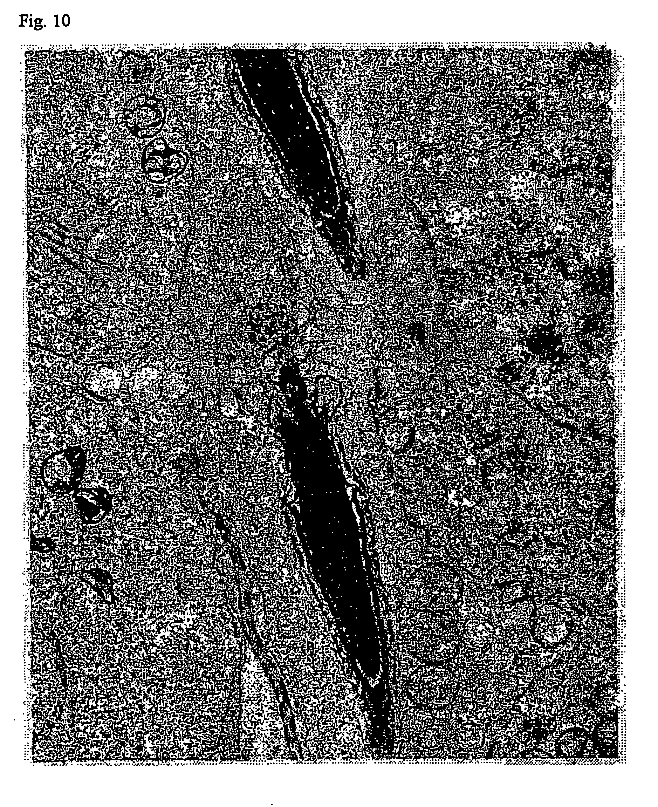

Formation of cytoplasmic vacuoles in Leydig's cells, swelling and denaturation of mitochondria, and decrease and deformation of smooth endoplasmic reticulum were observed in the group administered with TCDD alone. Increase of phagolysosomes and lipid granules, and nuclear condensation were also observed. In seminiferous tubules, separation between basement membrane and reproductive cells was characteristically found, and irregular forms of nuclei in reproductive cells and chromatin condensation were also observed. Further, disruption and loss of cytoplasmic organelles were found. By contrast, in the group administered with UDCA, in almost all cases, similar cellular form and microstructure to those of the solvent control were retained and in some cases, cellular microstructure was well developed (see Figs 4 to 10).

### 9) Determination of Johnsen's score for quantitation of histopathological findings

**Table 11.**

| Johnsen's score of the testis tissue | | |
|---|---|---|
| Dose of TCDD(µg/kg) | Additive contained in feed(wt%) | Score |
| 0.0 | Solvent control | 9.22±0.07 |
| 6.9 | UDCA(0.000) | 8.06±0.09** |
| | UDCA(0.125) | 8.7±0.09## |
| | UDCA,(0.250) | 8.84±0.10## |
| | UDCA(0.500) | 9.10±0.08## |
| 27.5 | UDCA(0.000) | 7.42±0.11** |
| | UDCA(0.125) | 8.40±0.10## |
| | UDCA(0.250) | 8.42±0.08## |
| | UDCA(0:500) | 8.81±0.07## |

| | | |
|---|---|---|
| **: Significant difference from the solvent control in p<0.01 ##: Significant difference from the group administered with TCDD alone in p<0.01 | | |

As shown in the above Table 11, in the each group administered with the dose of TCDD 6.9µg/kg alone and 27.5 µg/kg alone, statistically significant difference (p<0.01) was observed compared with the solvent control. Statistically significant difference (p<0.01) was observed between the group administered with the dose of TCDD 6.9 µg/kg alone and the group co-administered with the dose of TCDD 6.9 µg/kg, and 0.125, 0.25 and 0.5% by weight of UDCA, respectively. In addition, statistically significant difference (p<0.01) was observed between the group administered with the dose of TCDD 27.5 µg/kg alone and the group co-administered with the dose of TCDD 27.5 µg/kg, and 0.125, 0.25 and 0.5% by weight of UDCA, respectively. Thus, blocking effect of UDCA on TCDD toxicity was demonstrated. Further, such effects were increased dose-dependently in both groups administered with the dose of TCDD 6.9 µg/kg and 27.5 µg/kg.

### Example 2: Effect of bisphenol A on uterine proliferation

### 1. Experimental animals and administration design

Ovariectomized female B6C3F1 mice of 5 weeks old, the average weight of which was 18 to 22 g and had been bred in CRJ, were purchased. They were acclimated under the constant breeding conditions, *i.e*. the constant temperature of 24±1 °C, the humidity of 60±10% and the light and darkness cycle of 12 hours, for one week and then, utilized in the experiment. The animals were supplied with solid feed purchased from Purina and supplied with water ad libitum. After acclimation, test materials dissolved in corn oil were subcutaneously injected once a day for 4 days. The test materials used herein, stock solutions of bisphenol A and UDCA was prepared using ethanol. 2 mg of bisphenol A dissolved in 0.2 ml of corn oil was administered to the mice alone or in combination with UDCA once a day. The administration dose of UDCA was 0.2 or 2.0 mg, and corn oil was administered to the solvent control.

### 2. Measurement of the uterus weight

The experimental animals were observed for exhibition of clinical toxicity once a day and upon completion of the experiment, the uterus was extracted and weighed.

### 3. Statistical analysis

The results are expressed as Mean±Standard Deviation values. ANOVA was performed to evaluate the mean distribution of administered groups. In case that the significance was recognized in ANOVA, the Student's t-test was carried out in the levels of p<0.05 and p<0.01.

### 4. Results

**Table 12.**

| Effect of UDCA on changes in the uterus weight by bisphenol A | | |
|---|---|---|
| Administration | No. of test animals | Mean weight of uterus±SD |
| Control(corn oil) | 10 | 7.03±1.25 |
| Bisphenol A(2 mg/day) | 10 | 13.5±1.75** |
| Bisphenol A(2 mg/day) + UDCA(0.2 mg/day) | 10 | 11.74±1.36 |
| Bisphenol A(2 mg/day) + UDCA(2 mg/day) | 10 | 9.16±1.35## |
| UDCA(2 mg/day) | 10 | 6.96±1.34 |

| | | |
|---|---|---|
| **: Significant difference from the control (administered with corn oil) in p<0.01 ##: Significant difference from the group administered with the dose of bisphenol A 2 mg/day in p<0.01 | | |

As shown in the above Table 12, the weight of uterus was about two-fold increased by administration of 2 mg of bisphenol A once a day The co-administration of 0.2 mg of UDCA did not lead to any significant changes, but the co-administration of 2.0 mg of UDCA reduced the increased weight of uterus due to the administration of bisphenol A to that of the corn oil administered control.

In conclusion, bisphenol A, an exogenous estrogen, was confirmed to have the same physiological activity as estrogen *in vivo,* representatively exemplified by causing uterine proliferation which results in the increase of uterus weight. UDCA exhibited antagonistic activity against such effect.

### Formulation 1: Granules

| | |
|---|---|
| UDCA | 10 wt% |
| Lactose | 65 wt% |
| Starch | 19 wt% |
| Hydroxypropylcellulose | 5 wt% |
| Magnesium stearate | 1 wt% |

The above ingredients were mixed and the whole mixture was kneaded using 30 wt% of ethanol as a binding agent. Then, the kneaded mixture was granulated according to a conventional method, and then, dried. The granules were filled into packs at an amount of 1 g/pack, giving granules each containing 100 mg of dried UDCA.

### Formulation 2: Tablets

Granules prepared in the above Formulation 1 were compressed into tablets at an amount of 500 mg/tablet, giving tablets each containing 50 mg of UDCA, according to a conventional method.

### Formulation 3: Syrup

| | |
|---|---|
| UDCA | 0.6 wt% |
| Sucrose | 50 wt% |
| Sodium carboxymethylcellulose | 0.05 wt% |
| Methyl paraben | 0.08 wt% |
| Flavor | 0.1 wt% |
| Purified water | q. s. |

To 49.17 parts by weight of purified water was added sodium carboxymethylcellulose and then, swollen. Sucrose and methyl paraben were added to the resultant mixture to dissolve them by warming. To the resultant solution was added UDCA and the whole was stirred to give a homogenous solution. Flavor was added thereto and mixed. The resultant solution was set to a final volume of 100 ml and then, filtered. The filtrate was filled into bottles at a volume of 33 ml/bottle, giving syrup each containing 200 mg of UDCA.

### Formulation 4: Injections

| | |
|---|---|
| UDCA | 2 wt% |
| Ethyl alcohol | 2 wt% |
| Polyethylene glycol | 0.5 wt% |
| Sodium chloride | q. s. |

The above ingredients were adjusted to have pH 6.5 by addition of 2 M sodium hydroxide solution. Then, distilled water for injection was added thereto to a total volume of 100 ml.

### INDUSTRIAL APPLICABILITY

According to the present invention, UDCA has excellent efficacy to suppress toxicity caused by environmental hormones and thus, to prevent or treat any diseases caused by the exposure thereto, for example, weight loss, growth suppression, hypoglycemia, immunosuppression, reproductive toxicity, immunotoxicity, *etc*.

Further, UDCA suppresses endocrine disrupters by antagonistic activity against exogenous substances having estrogen-like activity.

## Claims

1. Formulations containing as an active ingredient ursodeoxycholic acid, or a sodium salt or taurine conjugate thereof, for use in a method of treating uterine hypertrophy caused by an environmental hormone having estrogen-like activity.

2. The formulations according to claim 1, wherein the environmental hormone is bisphenol A.

3. The formulations according to any of claims 1 to 2, further containing a pharmaceutically acceptable carrier.

4. The formulations according to claim 3, which are adapted for oral administration or for injection.

5. The formulations according to claim 4, which are adapted for muscular or intravenous injection.

## Patentansprüche

1. Formulierungen, enthaltend als einen Wirkstoff Ursodeoxycholsäure oder ein Natriumsalz oder Taurinkonjugat davon, zur Verwendung in einem Verfahren zur Behandlung von Hypertrophie der Gebärmutter, verursacht durch ein Umwelthormon mit Östrogen-artiger Aktivität.

2. Formulierungen nach Anspruch 1, wobei das Umwelthormon Bisphenol A ist.

3. Formulierungen nach einem beliebigen der Ansprüche 1 bis 2, ferner enthaltend einen pharmazeutisch verträglichen Träger.

4. Formulierungen nach Anspruch 3, die für eine orale Verabreichung oder zur Injektion adaptiert sind.

5. Formulierungen nach Anspruch 4, die zur muskulären oder intravenösen Injektion adaptiert sind.

## Revendications

1. Formulations contenant comme ingrédient actif de l'acide ursodésoxycholique, ou un sel de sodium ou un conjugué taurine de celui-ci, pour une utilisation dans un procédé de traitement de l'hypertrophie utérine provoquée par une hormone de l'environnement ayant une activité semblable à l'oestrogène.

2. Formulations selon la revendication 1, dans laquelle l'hormone de l'environnement est le bisphénol A.

3. Formulations selon l'une quelconque des revendications 1 à 2, contenant en outre un support pharmaceutiquement acceptable.

4. Formulations selon la revendication 3, qui sont adaptées pour une administration par voie orale ou pour une injection.

5. Formulations selon la revendication 4, qui sont adaptées pour une injection musculaire ou intraveineuse.
